# EUROPEAN PATENT APPLICATION

(11) **EP 4 834 923 A1**
(43) Date of publication of application: **04.11.2026**
(21) Application number: 25174087.4
(22) Date of filing: 02.05.2025
(51) Int. Cl.: C07K 14/575, A61K 38/00, A61K 38/22, A61P 3/04, A61P 3/10, C07K 14/585

(54) **AMYLIN RECEPTOR AGONIST PEPTIDE HORMONES**

(71) Applicant: Alveus Therapeutics Inc., Philadelphia, PA19103 (US)
(72) Inventor: The designation of the inventor has not yet been filed
(74) Representative: EIP

(57) **Abstract**

The present invention relates to novel peptide hormone analogues which are useful in treating disorders such as diabetes and obesity. The analogues are variants of islet amyloid polypeptide. The invention also encompasses methods for their production and their use in therapy, including the treatment, prevention, and/or amelioration of metabolic diseases, disorders, and/or conditions. The peptide hormone analogues may be conjugated to moieties which extend the plasma half-life such as serum albumin binding moieties, thereby providing long-acting agonists of amylin receptors.

## Description

### FIELD OF THE INVENTION

The present invention relates to novel peptide hormone analogues which are useful in treating disorders such as diabetes and obesity. The analogues are variants of islet amyloid polypeptide. The invention also encompasses methods for their production and their use in therapy, including the treatment, prevention, and/or amelioration of metabolic diseases, disorders, and/or conditions. The peptide hormone analogues may be conjugated to moieties which extend the plasma half-life such as serum albumin binding moieties, thereby providing long-acting agonists of amylin receptors.

### BACKGROUND OF THE INVENTION

Obesity is a chronic disease characterized by excess adiposity that impairs health and affects about 800 million people worldwide [Eržen S et al. Int. J. Mol. Sci. 2024;25,1517; Wharton S et al. CMAJ. 2020;192,E875-91; Busetto L et al. Eur. J. Intern. Med. 2021;93,3-7]. It increases the risk for multiple metabolic complications including type 2 diabetes (T2D), metabolic-dysfunction associated steatotic liver disease (MASLD, previously known as non-alcoholic fatty liver disease, NAFLD), cardiovascular disease as well as many mechanical complications such as osteoarthritis and obstructive sleep apnea (OSA) [Kinlen D et al. QJM. 2018;111:437-443].

Obesity accounted for over 5 million deaths globally in 2019, with more than half of these deaths occurring among people under 70 years old [GBD 2019 Risk Factor Collaborators. Lancet 2020; 396:1223-49]. The rise in obesity prevalence has both health and economic consequences.

Whilst 5-10% weight loss is clinically beneficial, more efficacious therapies without adverse side effects are required for the individual to improve or achieve remission of obesity-related complications [Garvey T J. Clin. Endocrinol. Metab. 2022;107:E1339-47; Tahrani AA and Morton J. Obesity 2022;30:802-840].

Amylin, also known as islet amyloid polypeptide (IAPP), is a 37-amino-acid peptide (molecular mass of ~4 kDa) with an intramolecular disulfide bridge between cysteine residues at positions 2 and 7 and an amidated C-terminus. It shares approximately 50% of the amino acid sequence with calcitonin gene-related peptides (CGRPα and CGRP β) and is structurally related to calcitonin. Amylin is co-secreted with insulin from pancreatic beta cells. Its multiple actions and beneficial effects on glycemic control, gastric emptying, energy expenditure and satiety are well studied. Amylin binds to calcitonin (CTR) and amylin (AMYR1-3) receptors with high potency, binding primarily to the AMY1R and AMY3R receptors to exert its physiological and pharmacological effects [Eržen S et al. Int. J. Mol. Sci. 2024;25,1517].

Amylin receptors are heterodimers of the calcitonin receptor (a G-protein coupled receptor), and one (or more) receptor-activity-modifying proteins (RAMPs), which are co-expressed in the same cell. Three RAMPs (RAMP1, RAMP2 and RAMP3) have so far been defined as relevant in the amylin receptor family, resulting in the known AMY1R, AMY2R and AMY3R receptors. Data supports that the AMY3R receptor may be particularly relevant for amylin's effects on eating and glucose regulation and that AMY1R may be particularly relevant for the control of fat utilization and energy expenditure [Coester B et al. Neuroscience 2020;447, 74-93; Lutz TA Appetite 2022;172,105965].

The first commercial amylin peptide analogue, Pramlintide, has a short action (~20-45 min half-life in humans) requiring multiple daily injections to be efficacious [Roth JD et al. Br. J. Pharmacol. 2012;166,121-136]. This drawback has been overcome by linking amylin peptides to plasma half-life extending moieties such as serum albumin binding moieties ("lipidation") and several long-acting amylin peptides are currently in clinical development for treatment of obesity [Melson E et al. Int. J. Obes. 2024 https://doi.org/10.1038/s41366-024-01473-y]. Pramlintide has A25P, S28P, S29P substitutions relative to IAPP, with the purpose of inhibiting peptide instability from aggregation and amyloid formation [Chi-cheng C et al. Biophys J 2013;105,1227-1235].

Another known amylin receptor agonist is NN1213, which has the sequence KCNTATCATQRLADFLRHSSPNFGAIPSSTNVGSRTY. NN1213 has N14D, V17R, N21P, L27P and N35R substitutions relative to IAPP, with the purpose of inhibiting peptide instability from aggregation and amyloid formation [Chi-cheng C et al. Biophys J 2013;105,1227-1235].

These amylin peptides are either dual acting CTR and AMYR agonists (DACRAs) or are moderately selective for AMYR1/3 over CTR. Recently, NN1213 and other long-acting selective AMYR1/3 vs CTR selective amylin peptides have been described showing robust effects on food intake and body weight lowering in animal models [Dahl K et al. J. Med. Chem. 2024;67,11688-11700] with readout from early clinical studies pending. A pramlintide variant with an S19K substitution has been disclosed (named San385) which is said to be selective for AMY3R over CTR (Cao J et al., Nat Chem Biol. 2024 Feb;20(2):162-169 https://doi.org/10.1038/s41589-023-01393-4).

The most advanced long-acting amylin peptide in clinical development is cagrilintide [Frias JP et al. Lancet 2023;402,P720-P730]. This is a dual amylin calcitonin receptor agonist (DACRA) with clinical meaningful body weight lowering efficacy but also significant adverse effects, most notably gastrointestinal side effects hampering patient acceptance and compliance. The present invention provides novel weight loss agents based on amylin peptide variants, and which are more selective towards amylin receptors (over the calcitonin receptor) than some known agonists.

### SUMMARY OF THE INVENTION

A first aspect of the invention provides an amylin 3 receptor (AMY3R) agonist, comprising a peptide with at least 91% sequence identity with Formula (Ib):

KCX₃TX₅TCX₈X₉X₁₀RLX₁₃DFLRX₁₈X₁₉SX₂₁X₂₂FGX₂₅X₂₆PX₂₈X₂₅TNVGSRTX₃₇ Formula (Ib)

wherein:
X₃ is selected from N and R;
X₅ is selected from A and G;
X₈ is selected from A and T;
X₉ is selected from T and V;
X₁₀ is selected from Q and G;
X₁₃ is selected from A and S;
X₁₈ is selected from H, K and R;
X₁₉ is selected from S and K;
X₂₁ is selected from N, P, K and Aib;
X₂₂ is selected from N, NMeN and K;
X₂₅ is selected from A and P;
X₂₆ is selected from I, NMel, N and K;
X₂₈ is selected from S, A and P;
X₂₉ is selected from S and P;
X₃₇ is selected from Y and pxF;
wherein at least one of:
- X₃ is R; and/or
- X₅ is G; and/or
- X₈ T; and/or
- X₉ is V; and/or
- X₁₀ is G; and/or
- X₁₃ is S; and/or
- X₁₈ is K or R; and/or
- X₁₉ is K; and/or
- X₂₁ is K or Aib; and/or
- X₂₂ is NMeN or K; and/or
- X₂₅ is P; and/or
- X₂₆ is NMel, N or K; and/or
- X₂₈ is A or P; and/or
- X₂₉ is P; and/or
- X₃₇ is pxF.

The inventors have surprisingly found that these agonists are more selective towards the amylin 3 receptor (AMY3R) over the calcitonin receptor (CTR), as compared to various known amylin receptor agonists. Specifically, these agonists are variants of NN1213, but which show improved selectivity for AMY3R over CTR as compared to NN1213.

In some cases, at least one of:
- X₁₈ is R or K; and/or
- X₁₉ is K; and/or
- X₂₁ is K or Aib; and/or
- X₂₂ is NMeN or K; and/or
- X₃₇ is pxF, such as azF, aaF or aeF.

In some cases, X₃ is N, X₅ is A; X₈ is A, X₉ is T, X₁₀ is Q, X₁₃ is A.

In some cases, X₂₅ is A, X₂₆ is I, X₂₈ is S and X₂₉ is S.

In some cases, the peptide has at least 91% sequence identity with Formula (IIb):

KCNTATCATQRLADFLRX₁₈X₁₉SX₂₁X₂₂FGAIPSSTNVGSRTX₃₇ Formula (IIb).

In some cases, the peptide has at least 91% sequence identity with Formula (IIIb):

KCNTATCATQRLADFLRHX₁₉SX₂₁X₂₂FGAIPSSTNVGSRTX₃₇ Formula (Illb).

In some cases, X₁₉ is K and/or X₂₁ is K and/or X₂₂ is K. In some such cases, at least X₁₉ is K. In some cases, X₃₇ is pxF, such as azF, aaF or aeF.

In some cases, there is a covalent bridge between the sulphurs of the cysteines at the 2 and 7 positions. In some such cases, the sulphurs are bridged via a covalent bond or a linking unit, wherein the linking unit is C₁₋₃alkylene.

The inventors have surprisingly found that these agonists show improved are more selective towards amylin 3 receptors over calcitonin receptors, as compared to various known amylin receptor agonists. Specifically, these agonists are variants of NN1213, but which show improved selectivity for AMY3R over CTR as compared to NN1213.

The invention also provides such an amylin 3 receptor agonist (for example, comprising a peptide with at least 91% sequence identity with Formula (Ib)), and a fatty acid covalently attached to the peptide.

In some cases, the fatty acid is covalently attached to the lysine at position 1 or a lysine at positions 22 or 26 in the peptide. Suitably, the fatty acid may be covalently attached to the lysine at position 1 or a lysine at position 26 in the peptide. In particular cases, the fatty acid is covalently attached to side chain of the lysine at position 1 in the peptide.

In some cases, the fatty acid is a C₁₄₋₂₂ diacid. In some cases, the fatty acid is covalently attached via a linker. In some cases, the fatty acid and linker together are selected from: 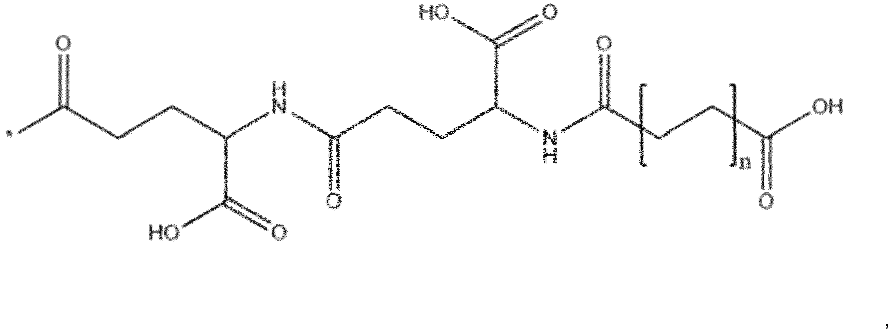 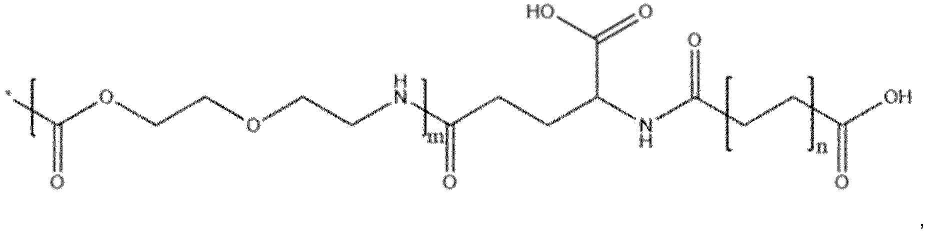 and 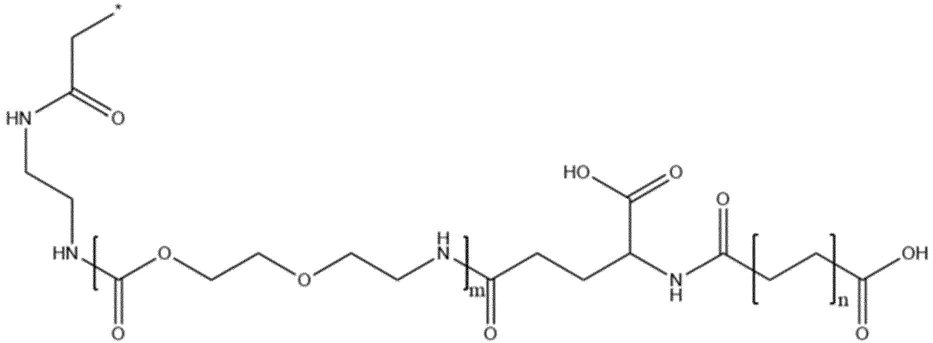 wherein * indicates the point of attachment to the nitrogen in the amino side chain of the lysine n is selected from 12, 14, 16, 18 and 20, and m is 2.

In some cases, two or more fatty acids are covalently attached to respective amino acids in the peptide. Suitably, in such cases, the fatty acids may be monoacids, each optionally covalently attached via linkers.

A second aspect of the invention provides a pharmaceutical composition comprising an amylin 3 receptor agonist according to the first aspect, and a pharmaceutically acceptable excipient.

A third aspect of the invention provides an amylin 3 receptor agonist according to the first aspect or a pharmaceutical composition according to the second aspect, for use in therapy. Also provided is a method of treating or preventing a metabolic disease, disorder and/or condition in a subject, comprising administering to the subject an amylin 3 receptor agonist according to the first aspect or a pharmaceutical composition according to the second aspect.

A further aspect of the invention provides an amylin 3 receptor agonist according to the first aspect or a pharmaceutical composition according to the second aspect, for use in treating or preventing a metabolic disease, disorder and/or condition. Such use may be, in some cases, in the treatment or prevention of obesity.

Also provided is the use of an amylin 3 receptor agonist according to the first aspect or a pharmaceutical composition according to the second aspect, the manufacture of a medicament for the treatment or prevention of a metabolic disease, disorder and/or condition, such as obesity.

A further aspect of the invention provides for non-therapeutic use (e.g. cosmetic use) of an amylin 3 receptor agonist according to the first aspect or a pharmaceutical composition according to the second aspect, in a method of weight loss, the method comprising administering the amylin 3 receptor agonist or composition to a subject.

### BRIEF DESCRIPTION OF SEQUENCE LISTING

**Table 1: Compound Sequence Listing**

| | |
|---|---|
| SEQ ID NO: 1 IAPP | K[CNTATC]ATQ RLANFLVHSS NNFGAILSST NVGSNTY |
| SEQ ID NO: 2 NN1213 | K(γE-γE-C20diacid)[CNTATC]ATQ RLADFLRHSS PNFGAIPSST NVGSRTY |
| SEQ ID NO: 3 | K[CNTATC]ATQRLADFLRHSSKNFGAIPSSTNVGSRTY |
| SEQ ID NO: 4 | K[CNTATC]ATQRLADFLRHKSKNFGAIPSSTNVGSRTY |
| SEQ ID NO: 5 | K[CNTATC]ATQRLADFLRHKSKKFGAIPSSTNVGSRTY |
| SEQ ID NO: 6 | K[CNTATC]ATQRLADFLRHKSPKFGAIPSSTNVGSRTY |
| SEQ ID NO: 7 | K(γE-γE- C20diacid)[CNTATC]ATQRLADFLRHKSKKFGAIPSSTNVGSRTY- amide |
| SEQ ID NO: 8 | K(γE-γE- C20diacid)[CNTATC]ATQRLADFLRHKSKNFGAIPSSTNVGSRTY- amide |
| SEQ ID NO: 9 | K[CNTATC]ATQRLADFLRHKSKKFGAIPSSTNVGSRTaeF-amide |
| SEQ ID NO: 10 | K(Oeg-Oeg-γE- C18diacid)[CNTATC]ATQRLADFLRHKSPNFGAIPSSTNVGSRTY- amide |
| SEQ ID NO: 11 | K(Oeg-Oeg-γE- C20diacid)[CNTATC]ATQRLADFLRHKSPNFGAIPSSTNVGSRTY- amide |
| SEQ ID NO: 12 | K(Oeg-Oeg-γE- C18diacid)[CNTATC]ATQRLADFLRHSSKNFGAIPSSTNVGSRTY- amide |
| SEQ ID NO: 13 | K(Oeg-Oeg-γE- C20diacid)[CNTATC]ATQRLADFLRHSSKNFGAIPSSTNVGSRTY- amide |
| SEQ ID NO: 14 | K(Oeg-Oeg-γE- C18diacid)[CNTATC]ATQRLADFLRHSSPKFGAIPSSTNVGSRTY- amide |
| SEQ ID NO: 15 | K(Oeg-Oeg-γE- C20diacid)[CNTATC]ATQRLADFLRHSSPKFGAIPSSTNVGSRTY- amide |

The square bracket [ ] between the two cysteine residues (cys 2 and cys 7) indicate the presence of an intramolecular disulphide bridge.

In each case, fatty acid conjugation is to the nitrogen atom in the side chain.

### DETAILED DESCRIPTION

As will be discussed, the present invention relates to novel amylin peptide hormone analogues which show a greater selectivity for AMY3R agonism over CTR agonism, when compared to IAPP and other known amylin analogues including NN1213. The amylin variants of the invention retain AMY3R agonism with reduced CTR agonism, whilst having pharmaceutically acceptable EC₅₀ and half-life values.

Accordingly, in some cases, the amino acid substitutions relative to IAPP and NN1213 disclosed herein result in a reduction of CTR agonism (relative to IAPP and NN1213) alongside substantial retention (or a proportionally smaller reduction) of AM3YR agonism in a given cell line at a therapeutically effective dose. In some other cases, AMY3R agonism may be increased (relative to IAPP and NN1213), whilst CTR agonism increases by a proportionally smaller amount (or is similar or reduced relative to IAPP and NN1213) at a therapeutically effective dose.

The variants can also be derivatised. All features described herein in relation to derivatives are explicitly, directly, and unambiguously disclosed in combination with all of the features described herein in relation to variants, and vice versa. Also disclosed is a pharmaceutical composition comprising a variant (or derivative thereof) according to the invention. Also disclosed is the variant, derivative or pharmaceutical composition of the invention for use in a method of therapy, such as for treating or preventing a metabolic disease, disorder, and/or condition, such as obesity.

### DEFINITIONS

In what follows, Greek letters are represented by their symbol rather than their written name. For example, α = alpha, ε = epsilon, γ = gamma and µ = mu. Amino acid residues may be identified by their full name, three-letter code or one-letter code, all of which are fully equivalent.

The term "amylin" as used refers to the native 37 residue protein, also known as islet amyloid polypeptide (IAPP), which is secreted by the pancreatic β-cells. The sequence is KCNTATCATQRLANFLVHSSNNFGAILSSTNVGSNTY (SEQ ID NO: 1), and this may also be referred to herein as native, human or wild-type amylin (hAMY).

All sequences are written from the N-terminal to the C-terminal, as is conventional.

The term "variant" as used herein refers to a protein based on amylin in which one or more amino acid substitutions, deletions, and insertions, or combinations thereof, are present as compared to the native amylin protein.

The term "substitution", as used herein, can mean the replacement of an amino acid in the amylin protein sequence with another. A substitution may be a conservative or a non-conservative substitution. The substitution herein may comprise the replacement of an amino acid from the native IL-22 sequence with a naturally occurring amino acid (a "natural amino acid") or a synthetic amino acid (a "non-natural amino acid"). In some preferred cases, the substitution comprises the replacement of an amino acid from the amylin sequence with a natural amino acid. Substitutions are defined herein relative to the amylin sequence; in other words, the site of substitution in the native protein is identified, and the replacement amino acid is indicated also. For example, a lysine (Lys, K) substitution for the native serine (Ser, S) at the 19 position of amylin is represented herein as "S19K" - which, alternatively expressed, means X₁₉ is K. Throughout this specification, where XiZ substitution notation is employed (where X is the IAPP amino acid, i is the site, and Z is the replacement amino acid), this can alternatively be expressed as Xᵢ is Z. These two different ways to indicate the amino acid at site Xᵢ are used interchangeably and are entirely equivalent to one another.

The term "deletion" as used herein, can mean the removal of an amino acid relative to the native amylin protein. The term "insertion", as used herein, can mean the addition of an amino acid relative to the native amylin protein. The term "extension", as used herein, can mean the addition of one or more amino acids to the N- or C-terminal of the native amylin protein or variant thereof, as disclosed herein.

The term "derivative" as sued herein, refers to an amylin variant protein as described herein, which is conjugated to one or more moieties which extend the plasma half-life, such as serum albumin binding moieties. In some cases, the amylin variant protein may be conjugated to one or more fatty acid, suitably by covalent attachment fatty acid. The term derivative encompasses both derivatives in which the fatty acid is covalently attached to the amylin variant protein directly and those in which the covalent attachment is by a linker.

The term "EC₅₀", as used herein, refers to the concentration of drug required to produce 50% of the maximal effect in a given assay. Potency is the amount of drug needed to produce a certain response. It depends on the affinity of the drug to the binding site, as well as the efficacy it can elicit. The less potent a drug is, the higher the dose needed to reach its effect. The half maximal effective concentration (EC₅₀) value is often used as a measure of the agonist potency at a given receptor, where a lower EC₅₀ value indicates a higher potency.

The term "half life" as used herein refers to the time taken for the concentration of a polypeptide in plasma to decline to 50% of its original level. Methods to determine the half-life of proteins are known in the art.

The claimed variants and derivatives exhibit a greater selectivity for amylin-3 receptor (AMY3R) agonism than for calcitonin receptor (CTR) agonism, as compared to known amylin variants and derivatives respectively (such as NN1213). As used herein, reference to "selectivity" or the like in the context of a variant, means that the response induced by that variant is biased. This can be quantified by the ratio of EC₅₀ values for the respective receptors. In other words, the claimed variants and derivatives have a larger Eₛₑₗ value than known variants and derivatives respectively: ${E}_{sel} = {EC}_{50} \left(CTR\right) / {EC}_{50} \left(AMY3R\right) .$

That is, the ratio of the EC₅₀(CTR) to EC₅₀(AMY3R) values increases with selectivity for AMY3R over CTR agonism, such that at a given drug concentration, a greater agonism response is induced in the amylin-3 receptor than the calcitonin receptor (as compared to known variants and derivatives).

The terms "naturally occurring amino acid" or "natural amino acid" refer to the group of amino acids consisting of: Arginine, Histidine, Lysine, Aspartic Acid, Glutamic Acid, Serine, Threonine, Asparagine, Glutamine, Cysteine, Selenocysteine, Glycine, Proline, Alanine, Valine, Isoleucine, Leucine, Methionine, Phenylalanine, Tyrosine, Tryptophan. Throughout this specification, these amino acids may be referred to by their conventional three-letter or single-letter abbreviations (e.g. Ala or A for alanine, Arg or R for arginine, etc.).

The 20 natural amino acids are typically classified into (i) charged amino acids, (ii) polar, uncharged amino acids, (iii) hydrophobic amino acids, and (iv) special case amino acids. Specifically, Arginine, Histidine, Lysine, Aspartic Acid, and Glutamic acid are known as "charged amino acids", with Arginine, Histidine and Lysine being positively charged. Serine, Threonine, Asparagine and Glutamine are known as "polar, uncharged amino acids"; of these, Asparagine and Glutamine are considered "large, polar, uncharged amino acids", each having 5 carbons in the backbone. Cysteine, Selenocysteine, Glycine and Proline are known as "special case amino acids". Alanine, Valine, Isoleucine, Leucine, Methionine, Phenylalanine, Tyrosine and Tryptophan are known as "hydrophobic amino acids"; or these, Methionine, Phenylalanine, Tyrosine and Tryptophan are considered "large hydrophobic amino acids", having sulphide or aromatic groups in the side chain.

Conservative amino acid substitutions are the exchange of an amino acid for another in the same class (e.g. substituting a hydrophobic amino acid for another hydrophobic amino acid). Non-conservative substitutions are the exchange of an amino acid for another in a different class (e.g. substituting a hydrophobic amino acid for a charged amino acid).

The terms "non-naturally occurring amino acid", "synthetic amino acid" or "non-natural amino acid" as used herein, refer to amino acids suitable for use in pharmaceutical peptides or proteins, which are not selected from the group of naturally occurring amino acids. "Non-natural amino acids" are often used in the development of pharmaceutical peptides or proteins and may be used in place of, or in addition to, the naturally occurring amino acids already present in the amino acid sequence of a given peptide or protein. Such "non-natural amino acids" are often used to achieve specific function in addition to their function as amino acid per se, e.g., to utilize non-proteinogenic or other stabilizing properties. "Non-natural amino acids" are commonly known to include (but are not limited to) D-amino acids, β-amino acids (denoted β-X herein, where X is the single-letter code for the corresponding natural amino acid), N-methyl amino acids (denoted NMeX herein, where X is the single-letter code for the corresponding natural amino acid), α,α-disubstituted amino acids, unnatural aromatic amino acids (e.g., D-phenylalanine), norleucine, hydroxyproline, phosphoserine, 4-fluorophenylalanine, azidohomoalanine. Specific examples of often used "non-natural amino acids" in the development of pharmaceutical peptides or protein drug candidates are selected from the group consisting of: Aib (a-aminoisobutyric acid, also referred to as 2-amino-2-methylpropanoic acid), Boc-lysine (t-butoxycarbonyl), Fmoc-lysine (9-fluorenylmethyloxycarbonyl), homoserine, 4-aminophenylalanine, αMeSer ((S)-2-amino-2-methyl-propanoic acid), αMePhe ((S)-2-amino-2-methyl-3-phenylpropanoic acid), Dab (2,4-diaminobutanoic acid), γ-Glu (γ-glutamic acid) and beta-alanine.

Another non-natural amino acid discussed herein is derived from phenylalanine; the side chain phenyl group has a para-substituent. This amino acid is referred to herein as pxF (para-X phenylalanine): 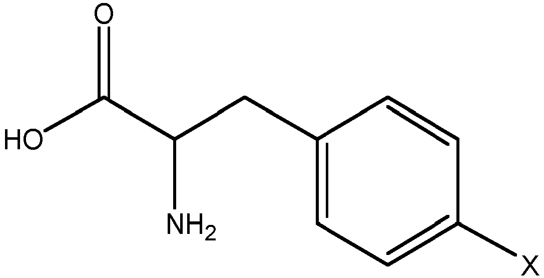 where X is selected from -NR₂, -NHC(=O)NR₂, NO₂, N-morpholino, methyl, ethyl, ethenyl or -Z-C₁-₄alkyene-NR₂, where Z is selected from CH₂, NH, O, S(=O) and S(=O)₂, and wherein each R is independently selected from H, methyl and ethyl.

In some cases, the side chain phenyl group has a para-[amino-linker] substituent. This amino acid is referred to herein as azF: 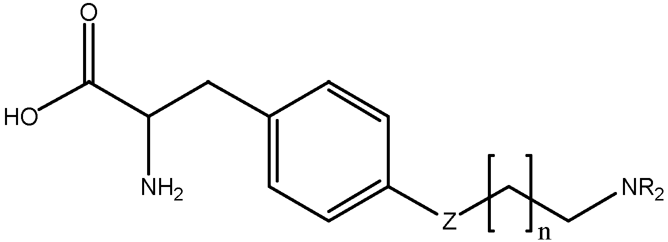 where Z is selected from CH₂, NH, O, S(=O) and S(=O)₂, n is selected from 0, 1, 2, 3, and each R is independently selected from H, methyl and ethyl.

In some cases, this amino acid has a para-aminoalkoxy substituent and is referred to as aaF [aminoalkoxyphenylalanine, also referred to as Phe(4-(2-aminoalkoxy)) and F(4-2aa)] and has the structure: 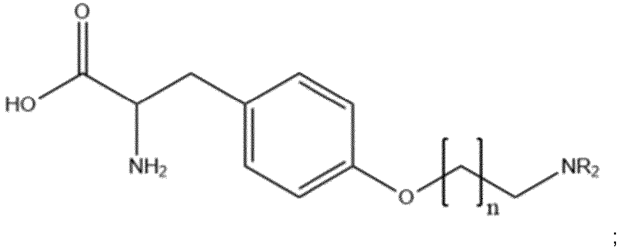 where n is selected from 0, 1, 2, 3, and each R is independently selected from H, methyl and ethyl.

In one particular case, the side chain phenyl group has a para-aminoethoxy substituent. This amino acid is referred to herein as aeF [aminoethoxyphenylalanine, also referred to as Phe(4-(2-aminoethoxy)) and F(4-2ea)] and has the structure:

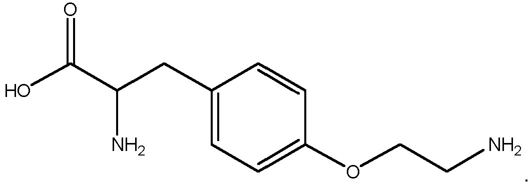

A "therapeutically effective amount" of a variant of the invention is any amount which, when administered to a subject, is the amount of variant that is needed to treat the disease, disorder and/or condition or produce the desired effect.

A "pharmaceutically acceptable vehicle" as referred to herein, is any known compound or combination of known compounds that are known to those skilled in the art to be useful in formulating pharmaceutical compositions.

Terms such as "treating" and "therapy", as used herein, expressly include the treatment, amelioration and/or prevention of a disease, disorder, and/or condition.

A "subject" or "patient", as used herein, may be a vertebrate, mammal, or domestic animal. Hence, variants and compositions according to the invention may be used to treat any mammal, for example livestock (for example, a horse), pets, or may be used in other veterinary applications. In some cases, the subject or patient may be human.

### Variants

The invention provides an AMY3R receptor agonist having at least 91% sequency identity with Formula (Ib). This means that there may be variations at up to 3 amino acids sites compared to that indicated in the Formula. In some cases, the agonist may have at least 94%, at least 97% or 100% sequence identity with Formula (Ib) (corresponding to 2, 1 and 0 differences respectively as compared to Formula (Ib)). Such variations may comprise deletions, insertions or substitutions, for example. In some cases, the variations are only substitutions.

The variants can, in some cases, also include N-terminal and/or C-terminal extensions to the structure of Formula (1b). Such extensions do not change the sequence identity. In some cases, the C-terminal may be linked to an amine group, such that the C-terminal concludes in an amide structure. The amine group may be NR₂ wherein each R is independently H or C₁₋₆alkyl.

The inventors have found that AMY3R receptor agonists with a substitution (relative to SEQ ID NO:2 - NN1213) at positions 3, 5, 9, 10, 13, 17-19, 21, 22, 25, 26, 28, 29 and 37 are more selective for AMY3R agonism over CTR agonism than NN1213 (and IAPP).

In particular, the following substitutions relative to NN1213 have been found to increase the peptide selectivity for AMY3R agonism over CTR agonism: H18R, H18K, S19K, P21K, N22K, Y37pxF (i.e. X₁₈ is R, X₁₈ is K, X₁₉ is K, X₂₁ is K, X₂₂ is K, X₃₇ is aaF), and in some cases, the pxF may be azF, aaF or aeF (i.e. Y37azF or X₃₇ is azF, Y37aaF or X₃₇ is aaF, such as Y37aeF or X₃₇ is aeF).

In particular, introduction of amino acids which differ from the NN1213 amino acids at positions 18, 19, 21, 22, and 37 have been found by the inventors to result in strong selectivity for AMY3R agonism over CTR agonism - a high Eₛₑₗ value.

Any and all combinations of the options in Formula Ib are disclosed. In some particular cases, X₁₉ is K. In some particular cases, X₂₁ is K. In some particular cases, X₂₂ is K. In some particular cases, X₃₇ is pxF, such as azF, aaF or aeF.

Some variants may comprise the following combinations of substitutions (relative to IAPP). Some variants may include the following combinations of substitutions, and optionally up to three further conservative substitutions. Some variants may include only the following combinations of substitutions (relative to IAPP):
(i) N14D, V17R, N21K, L27P, N35R; (ii) N14D, V17R, S19K, N21K, L27P, N35R; (iii) N14D, V17R, S19K, N21K, N22K, L27P, N35R; (iv) N14D, V17R, S19K, N21P, N22K, L27P, N35R; (v) N14D, V17R, S19K, N21K, N22K, L27P, N35R; (vi) N14D, V17R, S19K, N21K, L27P, N35R; (vii) N14D, V17R, S19K, N21K, N22K, L27P, N35R, Y37aeF; (viii) N14D, V17R, S19K, N21P, L27P, N35R; (ix) N14D, V17R, N21K, L27P, N35R.

Any and all variants disclosed herein can be derivatised.

### Derivatisation

The variants of the invention may be derivatized. The derivatisation comprises conjugation to moieties which extend the plasma half-life such as serum albumin binding moieties. The albumin binding moiety is any compound that binds to albumin. Exemplary albumin binding moieties suitable for use in the polypeptides of the invention include lipids (e.g. a fatty acid derivative), albumin-binding peptides, albumin binding proteins, or small molecule ligands that bind to albumin. In some cases, the amylin variant protein of the invention may be conjugated (e.g. covalently attached) to one or more fatty acid.

The term derivative encompasses both derivatives in which the fatty acid is covalently attached to the amylin variant protein directly and those in which the covalent attachment is by a linker.

Renal clearance of amylin and variants thereof is generally quite fast, limiting clinical use in practice due to consequent need for a high administration frequency. Conjugation of one or more lipids, such as fatty acids, enables binding to albumin, thereby preventing renal excretion. This conjugation may also provide some steric protection against proteolysis.

The derivatives of the invention may comprise one or more albumin binding moiety (e.g. lipid), such as one, two or three albumin binding moieties. In some cases, the derivatives comprise only one albumin binding moiety (e.g. lipid).

The albumin binding moiety (e.g. lipid) may be attached to an amino acid residue of the polypeptide. In some embodiments, the albumin binding moiety (e.g. lipid) is attached to the amino acid residue through a linker. In alternative embodiments, the albumin binding moiety (e.g. lipid) is directly attached to the amino acid residue without an intervening linker. The albumin binding moiety (e.g. lipid) may be attached to the amino acid residue via an ester, a sulfonyl ester, a thioester, an amide, an amine or a sulphonamide. Accordingly, it will be understood that the albumin binding moiety (e.g. lipid) or the linker includes an acyl group, a sulphonyl group, an N atom, an O atom or an S atom which forms part of the ester, sulphonyl ester, thioester, amide, amine or sulphonamide. Optionally, an acyl group in the albumin binding moiety (e.g. lipid) or the linker forms part of an amide or ester with the amino acid residue. Accordingly, in some cases the albumin binding moiety (e.g. lipid) is attached, directly or via a linker) to an acylation site on the amino acid residue.

The albumin binding moiety (e.g. lipid) may be attached to any residue of the polypeptide. This may be a residue present in the native amylin sequence, or a substituted residue (relative to the native amylin). In some embodiments, the albumin binding moiety (e.g. lipid) is attached to the side chain of an amino acid residue in the polypeptide. In some embodiments, the albumin binding moiety (e.g. lipid) is attached to the N-terminus of the polypeptide, (e.g. to a lysine at the N-terminus of the polypeptide).

In some cases, an albumin binding moiety is attached to a lysine at position 1 (N-terminus or side chain), 22 (side chain) or 26 (side chain). In some cases, an albumin binding moiety is attached to a lysine at position 1 (N-terminus or side chain) or 26 (side chain). In some cases, an albumin binding moiety is attached to the side chain of the lysine at position 1 (i.e. at the ε-N of the lysine).

In preferred embodiments, the albumin binding moiety is a lipid. Accordingly, the derivatives of the invention may comprise at least one lipid (referred to herein as "lipidated polypeptide"). Without being bound by theory, it is thought that the lipid acts as an albumin binding moiety and protects the polypeptide against clearance and degradation, thereby extending the half-life of the polypeptide.

In embodiments of any aspect of the invention, the lipid may comprise a hydrocarbon chain having from 10 to 26 C atoms, e.g. from 14 to 24 C atoms, e.g. from 16 to 22 C atoms. For example, the hydrocarbon chain may contain 14, 15, 16, 17, 18, 19, 20, 21, 22, 23 or 24 C atoms. In preferred cases, the lipid has 18 to 20 C atoms. In particular, the lipid may have 18 C atoms or 20 C atoms. The hydrocarbon chain may be linear or branched, and may be saturated or unsaturated. Furthermore, it can include a functional group at the end of the lipophilic chain, e.g. a carboxylic acid group which may or may not be protected during synthesis.

Optionally, the lipid comprises a dicarboxylic acid. For example, the lipid may comprise C₁₂diacid, C₁₄diacid, C₁₆diacid, C₁₇diacid, C₁₈diacid, C₁₉diacid or C₂₀diacid. In preferred embodiments, the lipid comprises C₁₈diacid or C₂₀diacid.

Where the albumin binding moiety is attached to the variant by a linker, the linker may comprise one or more residues of any naturally occurring or non-naturally occurring amino acid. The linker may comprise a combination of residues, as single or repeating units. For example, the linker may comprise multiple combinations of residues, as single or repeating units, each of which may independently be a residue of Glu, γ-Glu, Lys, ε-Lys, Asp, β-Asp, Gaba, β-Ala (3-aminopropanoyl), O2Oc (2-(2-(2-aminoethoxy)ethoxy)acetic acid) also known as OEG, PEG2 (3-(2-(2- aminoethoxy)ethoxy)propanoic acid), PEG4 (1-amino-3,6,9,12-tetraoxapentadecan-15-oic acid), PEG8 (1-amino-3,6,9,12,15,18,21 ,24-octaoxaheptacosan-27-oic acid, PEG12 (1 -amino-3,6,9, 12, 15, 18,21 ,24,27,30,33,36-dodecaoxanonatriacontan-39-oic acid). γ-Glu and β-Asp refer to amino acids where the alpha-amino group and the side chain carboxyl group participate in peptide bond formation. ε -Lys refers to an amino acid where the epsilon-amino and carboxyl group of lysine participate in peptide bond formation.

In some embodiments, the linker comprises a residue of γ-Glu. In some cases, the linker may selected from (i) γGlu, (ii) γGlu-γGlu, (iii) γGlu-(O2Oc)-(O2Oc)*, (iv) γGlu-(PEG2)-(PEG2)*, and (v) γGlu-(O2Oc)-(O2Oc)-NH(CH₂)₂NHCO-CH₂-*, wherein * in each case indicates the end of the linker attached to the polypeptide.

In some particular cases, the wherein the fatty acid and linker together are selected from: 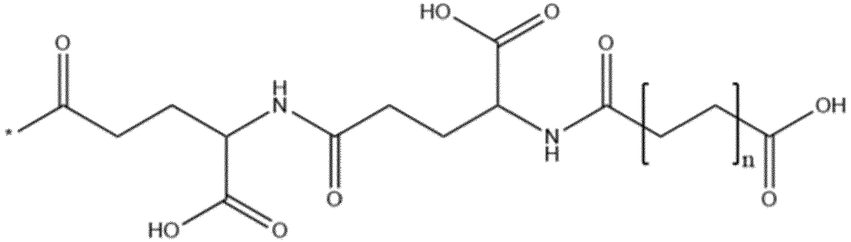 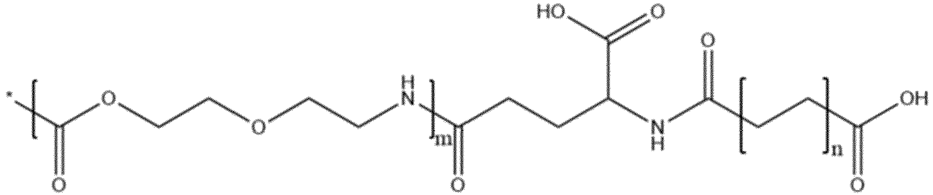 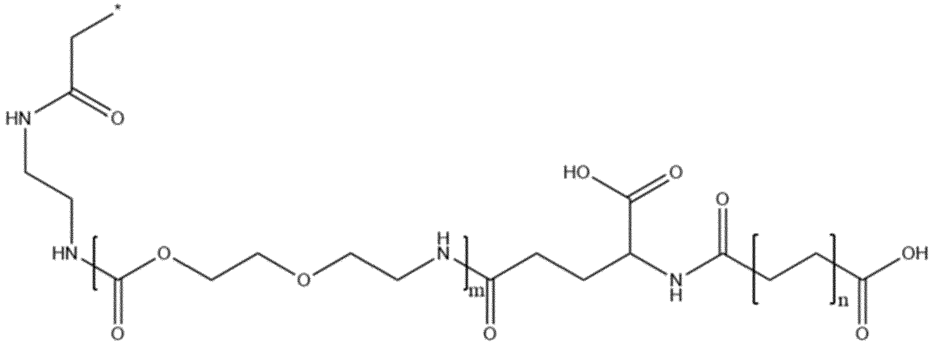 wherein * indicates the point of attachment to the nitrogen of the polypeptide, n is selected from 12, 14, 16, 18 and 20, and m is 2. Suitably, * indicates the point of attachment to the nitrogen in the amino side chain of the lysine (i.e. the (εN)K).

The derivatives of the invention (e.g. lipidated amylin variants) may have a half-life of at least 5 hours, at least 6 hours, at least 7 hours, at least 8 hours, at least 9 hours, at least 10 hours, at least 11 hours, at least 12 hours, at least 13 hours or at least 14 hours in rat models.

It will be recognised that an extended half-life is advantageous, as it permits the therapeutic proteins to be administered according to a safe and convenient dosing schedule, e.g. lower doses that can be administered less frequently. Moreover, the provision of lower doses may provide further advantages such an improved safety profile. To the contrary, pramlintide requires frequent and inconvenient administration.

### Calculating Percentage Identity

The skilled technician will appreciate how to calculate the percentage identity between two amino acid sequences. An alignment of the two sequences must first be prepared, followed by calculation of the sequence identity value. The percentage identity for two sequences may take different values depending on: (i) the method used to align the sequences, for example, ClustalW, BLAST, FASTA, Smith-Waterman (implemented in different programs), or structural alignment from 3D comparison; and (ii) the parameters used by the alignment method, for example, local versus global alignment, the pair-score matrix used (for example, BLOSUM62, PAM250, Gonnet etc.) and gap-penalty, for example, functional form and constants.

Having made the alignment, there are various ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of amino acid sequences is a complex process. The popular multiple alignment program ClustalW [48,49] is a preferred way for generating multiple alignments of proteins in accordance with the invention. Suitable parameters for ClustalW may be as follows: For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula: Sequence Identity = (N/T)*100.

Alternative methods for identifying similar sequences will be known to those skilled in the art.

### Variant Preparation

In one example preparation method, peptides described herein, including variants of the invention, were synthesized using a Liberty Blue^{™} Automated microwave peptide synthesizer, on Fmoc-protected rink amide resin with a loading of 0.32 mmol/g.
- Asparagine, cysteine, glutamine and histidine were incorporated as their sidechain trityl (Trt) derivatives
- Lysine was incorporated as the sidechain tert-butyloxycarbonyl (Boc) derivative.
- Serine, threonine and tyrosine were incorporated as sidechain tert-butyl (tBu) ethers.
- Aspartate was incorporated as a sidechain OtBu ester.
- Arginine was incorporated as the sidechain 2,2,4,6,7- pentamethyldihydrobenzofuran-5-sulfonyl (Pbf) derivative.
- Boc-Lys (Fmoc) was incorporated when a subsequent chemical modification of the N- terminal lysine side chain was required.

The resin was first swollen in DMF/DCM (1:1) for 10 minutes at ambient temperature. N°-Fmoc deprotection was performed by treating resin with 20% piperidine in DMF at 90°C for 2 minutes, followed by washing with DMF. Fmoc amino acids were dissolved at 0.2M in dry DMF(4 eq.). Coupling chemistry was performed using Oxyma (ethyl cyano(hydroxyimino)acetate) as an activator base (1M in DMF, 4 eq.), DIC (N, N'-diisopropylcarbodiimide) as an activator (1M in DMF, 4 eq.) with microwave irradiation at 90°C for 4 minutes. If certain amino acids required double coupling, the cycle was repeated with fresh reagents to ensure complete coupling (in this case, Q-10^{th} R-11^{th}). The resin was washed 4x with DMF after N°-Fmoc deprotection and 3x after couplings.

After completion of sequence till Boc-Lys (Fmoc)-OH, the resin was transferred to a solid-phase peptide synthesis vessel. Fmoc-Glu-OtBu(γ-Glu) and 20-(tert-butoxy)-20-oxoicosanoic acid (C20 diacid) were coupled manually. The resin was first washed with DMF & DCM (3x). Coupling steps were performed in two stages using 3 eq. each of amino acids and HOBt and 4 eq of DIC (2h); followed by second cycle using 3 eq. of amino acids, 3 eq. of Oxyma and 4 eq. of DIC (1h) at room temperature. N°-Fmoc deprotection was performed by treating resin with 20% piperidine in DMF for 15 minutes. The resin was then washed thoroughly with DMF (3x) and DCM (3x) before cleavage.

Cleavage was performed using TFA/TIPS/DTT/H₂O/Thioanisole (90:2.5:2.5:2.5:2.5) (kept in ice bath for 15 minutes) for 3 hours at room temperature. The crude peptide was precipitated with cold diethyl ether, centrifuged, and purified using reverse-phase HPLC (RP-HPLC) with a gradient of acetonitrile/water containing 0.1% TFA. The final product was lyophilized and characterized by LC-MS and analytical HPLC.

For disulfide bridging, the lyophilized peptide was dissolved in 20% DMSO in water at a concentration of 1 mL/mg peptide and stirred at room temperature for 16 hours. The reaction was monitored via LC-MS to confirm disulfide bond formation. The final product was purified using RP-HPLC and lyophilized to obtain the desired oxidized peptide.

In another example preparation method, solid-phase peptide synthesis vessels were employed, with batch size of 0.25 mmol and resin loading of 0.32 mmol/g.
- Asparagine, cysteine, glutamine and histidine were incorporated as their sidechain trityl (Trt) derivatives.
- Lysine was incorporated as the sidechain tert-butyloxycarbonyl (Boc) derivative.
- Serine, threonine and tyrosine were incorporated as sidechain tert-butyl (tBu) ethers.
- Aspartate was incorporated as a sidechain OtBu ester.
- Arginine was incorporated as the sidechain 2,2,4,6,7- pentamethyldihydrobenzofuran-5-sulfonyl (Pbf) derivative.
- Boc-Lys (Fmoc) was incorporated when a subsequent chemical modification of the N- terminal lysine side chain was required.

The resin was first swollen in DMF/DCM (1:1) for 30 minutes. Deprotection was carried out with 20% piperidine in DMF for 10 minutes at room temperature with shaking, followed by thorough washing with DMF (3x) and DCM (3x). Generally, each amino acid (3 eq) was coupled using HOBt (5 eq.) and DIC (5 eq.) in DMF for 120 minutes with continuous shaking. If certain amino acids required double coupling, the cycle was repeated using fresh reagents. The resin was washed with DMF (3x) and DCM (3x) after each coupling cycle. (The exception to this is Fmoc-Glu-OtBu and 20-(tert-butoxy)-20-oxoicosanoic acid residues, which were coupled in two stages using 3 eq. of amino acids, 5 eq. of HOBt and 5 eq. of DIC (2h); followed by second cycle using 3 eq. each of amino acid, oxyma and and DIC at room temperature for an hour.)

After completion of the synthesis, resin was transferred to Spinwin^{™} conical bottom tube (50 ml centrifuge tubes). Global deprotection was performed using TFA/ TIPS/ DTT/ H₂O/ Thioanisole (90:2.5:2.5:2.5:2.5) (10 ml / 1gm of resin) at 0° C. After that, the resin was kept in ice bath for 30 minutes and 2.5 hours at room temperature in shaker. The cleavage mixture was collected by filtration and crude peptide was precipitated with cold diethyl ether and centrifuged. The supernatant was decanted, and process was repeated 2-3 times. The obtained crude peptide was purified using reverse-phase HPLC (RP-HPLC) with a gradient of acetonitrile/water containing 0.1% TFA. The final product was lyophilized and characterized by LC-MS and analytical HPLC.

For disulfide bridging, the lyophilized peptide was dissolved in 20% DMSO in water at a concentration of 1 mL/mg peptide and stirred at room temperature for 16 hours. The reaction was monitored via LC-MS to confirm disulfide bond formation. The final product was purified using RP-HPLC and lyophilized to obtain the desired oxidized peptide.

The above examples are examples only, and are not limiting on the invention. The amylin variant can be obtained by any means known in the art, such as by recombinant means. An experienced investigator in the field would be readily able to identify suitable nucleic acid sequences that encode the desired amylin variants.

### Identifying Selectivity for AMY3R agonism over CTR agonism

The variants and derivatives of the invention are amylin receptor agonists, i.e. they are capable of binding to, and inducing signalling by, one or more receptor complexes comprising hCTR and at least one of the human receptor activity modifying proteins designated hRAMP1, hRAMP2 and hRAMP3 (i.e. at any of the complexes designated hAMY1R, hAMY2R and hAMY3R, also referred to as human amylin receptors 1, 2 and 3 respectively). In some embodiments, a compound is considered an amylin receptor agonist if it has agonist activity at one or more of hAMYR1, hAMYR2 and hAMYR3. In some cases, the variants and derivatives of the invention are hAMY3R agonists.

The ability to induce cyclic adenosine monophosphate (cAMP) formation as a result of binding to the relevant receptor or receptor complex is typically regarded as indicative of agonist activity. Other intracellular signaling pathways or events may also be used as readouts for amylin receptor agonist activity. These may include calcium release, β-arrestin recruitment, receptor internalization, kinase activation or inactivation, lipase activation, inositol phosphate release, diacylglycerol release or nuclear transcription factor translocation.

As noted above, the amylin variants and derivatives of the present invention are selective for AMY3R agonism over CTR agonism. This selectivity means that the response induced the amylin variant or derivative is biased; it induces a proportionally greater AMY3R agonism response than CTR agonism response, as compared to native amylin, and other known amylin variants such as pramlintide, San385 and NN1213.

The claimed variants and derivatives have a larger Eₛₑₗ value than known variants and derivatives respectively: ${E}_{sel} = {EC}_{50} \left(CTR\right) / {EC}_{50} \left(AMY3R\right) .$

That is, the ratio of the EC₅₀(CTR) to EC₅₀(AMY3R) values increases with selectivity for AMY3R over CTR agonism, such that at a given drug concentration, a greater agonism response is induced in the amylin-3 receptor than the calcitonin receptor. The variants and derivatives of the invention are full agonists - that is, they have a similar maximum antagonistic effect for AMY3R and CTR receptors as IAPP/hAmylin.

Identifying selectivity involves consideration EC₅₀ values for the variant or derivative in a cell line. EC₅₀ values can be determined by assays known in the art, and some such assays are described in detail in the Examples section below. When comparing EC₅₀ values for a variant or derivative of the invention, and IAPP or known variants/derivatives, it is only important that the same methodology be used to determine the values for the polypeptide structures under comparison.

In some cases, the variants or derivatives of the invention have an Eₛₑₗ value (measured under the conditions described below in Example 1) of at least 20, 25, 30, 35 or 40. In some cases, the Eₛₑₗ is at least 30, at least 35, or at least 40. In some cases, the Eₛₑₗ is at least 70.

In some embodiments, the isolated polypeptide has an EC₅₀ for AMY3R measured under the conditions described below in Example 1 of below about 1 nM, below about 0.8 nM, below about 0.6 nM, below about 0.4nM, below about 0.3 nM, below about 0.2 nM or below about 0.1 nM.

### Pharmaceutical Composition

Therefore, according to a further aspect of the invention, there is provided a pharmaceutical composition comprising a variant (or derivative thereof) as described herein and a pharmaceutically acceptable excipient. The formulation of pharmaceutically active ingredients with various excipients is known in the art.

The pharmaceutical composition may be suitable for oral administration, or administration by inhalation or injection. Injection may be intraperitoneal, subcutaneous or intravenous, as further described herein.

A pharmaceutical composition of invention may comprise any of the different variants of amylin (or a derivative thereof) according to the invention.

In some cases, the pharmaceutical composition may be a solid; optionally the composition may be in the form of a powder for resuspension. The composition may include one or more substances which may also act as flavouring agents, lubricants, solubilisers, suspending agents, dyes, fillers, glidants, inert binders, preservatives, or dyes. The composition may encapsulate the variant or derivative.

In some cases, the pharmaceutical composition may be a liquid; optionally the pharmaceutical composition is in the form of a solution. Liquid vehicles are used in preparing solutions, suspensions, emulsions, syrups, elixirs, and pressurised compositions. The variant or derivative according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilisers, or osmo-regulators. Suitable examples of liquid vehicles for parenteral administration include water (partially containing additives as above, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil).

The process for preparing a pharmaceutical composition of the invention may thus comprise the usual steps that are standard in the art.

### Use in Therapy and Cosmetic treatment

According to a further aspect of the invention, there is provided a variant (or derivative thereof) of the invention or a pharmaceutical composition of the invention, for use in therapy. A method of treating a subject with a variant (or derivative thereof) of the invention, or a pharmaceutical composition comprising the same, is also provided. Any of the different variants and derivatives which are part of the invention are expressly included in these aspects of the invention.

The variant (or derivative thereof) of amylin or a pharmaceutical composition comprising the same may be administered directly into a subject to be treated. The variant, derivative or pharmaceutical composition may be administered by any means, including by inhalation, by injection or orally. When administered by inhalation, it may be via the nose or the mouth. Preferably, the variant (or derivative thereof) or pharmaceutical composition is administered by injection, typically subcutaneously or intravenously.

Liquid pharmaceutical compositions, which are sterile solutions or suspensions, can be utilised by, for example, intramuscular, intrathecal, epidural, intraperitoneal and particularly subcutaneous or intravenous injection. The variant or derivative may be prepared as a sterile solid composition that may be dissolved or suspended at the time of administration using sterile water, saline or other appropriate sterile injectable medium.

Oral administration may be suitably via a tablet, a capsule or a liquid suspension or emulsion. Suitable examples of liquid vehicles for oral administration include water (partially containing additives, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). The variants and derivatives of the invention may be administered orally in the form of a sterile solution or suspension containing other solutes or suspending agents (for example, enough saline or glucose to make the solution isotonic), bile salts, acacia, gelatine, sorbitan monoleate, polysorbate 80 (oleate esters of sorbitol and its anhydrides copolymerized with ethylene oxide) and the like. Solutions, syrups, and elixirs also form part of the invention. The variants and derivatives according to the invention can also be administered orally in solid composition form. Solid compositions suitable for oral administration include pills, capsules, granules, tablets, and powders.

A variant, derivative or pharmaceutical composition of the invention may be administered to any subject in need thereof. Most preferably, the subject is a human being. The variants, derivatives and compositions need not only be administered to those already showing signs of a disease, disorder and/or condition. Rather, they can be administered to apparently healthy subjects as a purely preventative measure against the possibility of such a disease, disorder, and/or condition in future.

It will be appreciated that variants, derivatives and compositions according to the invention may be used in a monotherapy (i.e. the sole use of that variant, derivative or composition), for treating or preventing a disease, disorder, and/or condition. Alternatively, variants, derivatives and compositions according to the invention may be used as an adjunct to, or in combination with, known therapies for treating or preventing a disease, disorder, and/or condition.

It will be appreciated that the amount of the variant, derivative or composition that is required is determined by the biological activity, half-life, and bioavailability or the variant/derivative, which in turn depends on the mode of administration, the physiochemical properties of the variant/derivative and composition, and whether it is being used as a monotherapy or in a combined therapy. The frequency of administration will also be influenced by the half-life of the variant or derivative within the subject being treated.

Optimal dosages to be administered may be determined by those skilled in the art, and will vary with the particular variant or derivative in use, the strength of the pharmaceutical composition, the mode of administration, and the advancement of the disease, disorder and/or condition. Additional factors depending on the particular subject being treated will result in a need to adjust dosages, including subject age, weight, gender, diet and time of administration.

The invention also provides a variant, derivative or pharmaceutical composition of the invention for use in a method of treating or preventing a metabolic disease, disorder and/or condition, such as obesity. Methods of treating or preventing a metabolic disease, disorder and/or condition, such as obesity, using a variant, derivative or pharmaceutical composition of the invention are also provided.

The invention also provides for non-therapeutic use of a variant, derivative or pharmaceutical composition of the invention in a method of weight loss, comprising administering the variant, derivative or pharmaceutical composition to a subject.

There is no restriction on which variant or derivative or pharmaceutical composition of the invention should be administered to which patient (subject). Rather, it is intended that any of the variants and derivatives according to the invention and compositions containing the same as described herein can be administered to any patient (subject) as described herein.

All the features described herein (including any accompanying claims, abstract and drawings), and/or all the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made to the Examples, which are not intended to limit the invention in any way.

### EXAMPLES

### Example 1: Selectivity

The functional activation of human calcitonin receptor (hCTR) or human amylin 3 receptor (hAMY3R, formed with co-expression of calcitonin receptor and RAMP3 protein) on HEK293 cells transiently expressing the respective receptors was assayed measuring cyclic adenosine monophosphate (cAMP) production upon stimulation with peptides. HEK293 cells (1000 cells/well) were seeded overnight in a 384 well plate (Revvity, Cat. #6007480) and transfected with hCTR or hAMY3R receptor expressing plasmids (pcDNA 3.1 hCTR and pcDNA 3.1 hAMY3R) using Fugene HD (Promega, Cat. #E2311) transfection reagent. Total plasmid DNA of 50 ng was transfected per well using 0.15 µl of Fugene HD. The amount of CTR plasmid was kept constant for both CTR and AMY3R receptor wells.

48 hours post transfection, the media was replaced with stimulation buffer (1X HBSS (Invitrogen, cat. # 14025-092), 5 mM HEPES (Invitrogen, cat. # 15630-080), 0.5 mM IBMX (Sigma, cat. # I7018), 0.1% Ovalbumin (Sigma, Cat. #A5503), pH 7.4) and cells were stimulated with peptide analogues of amylin. Peptides were suspended in ultra-pure water and dilutions were prepared in the stimulation buffer. Cells were incubated with the diluted peptides (0.0001 - 100 nM) for 30 minutes at room temperature to generate a dose response curve.

Accumulation of cAMP upon stimulation was evaluated using a Lance Ultra cAMP kit (Revvity, Cat. #TRF0264). Post stimulation cells were lysed and cAMP levels were measured as per the manufacturer's recommendation. The plates were read on an Envision (Perkin Elmer) plate reader using an excitation wavelength of 320 nm and emission wavelengths of 620 nm and 665 nm.

Data was presented as FRET ratios and EC50s were generated from dose response curves (variable slope, 4-parameter) by non-linear regression fitting using Graphpad software. Selectivity towards the amylin receptor was calculated as: ${E}_{sel} = {EC}_{50} \left(CTR\right) / {EC}_{50} \left(AMY3R\right) .$

| SEQ ID NO: | Modifications (relative to IAPP - SEQ ID NO: 1) | EC₅₀(CTR) (nM) | EC₅₀(AMY3R) (nM) | Eₛₑₗ |
|---|---|---|---|---|
| 2 | N14D, V17R, N21P, L27P, N35R, 1K(γE-γE-C20diacid) | 5.06 | 0.26 | 19.5 |
| 3 | N14D, V17R, N21K, L27P, N35R | 0.726 | 0.026 | 27.9 |
| 4 | N14D, V17R, S19K, N21K, L27P, N35R | 3.016 | 0.105 | 28.7 |
| 5 | N14D, V17R, S19K, N21K, N22K, L27P, N35R | 5.57 | 0.22 | 25.3 |
| 6 | N14D, V17R, S19K, N21P, N22K, L27P, N35R | 3.32 | 0.13 | 25.5 |
| 7 | N14D, V17R, S19K, N21K, N22K, L27P, N35R, 1K(γE-γE-C20diacid) | 8 | 0.266 | 30.1 |
| 8 | N14D, V17R, S19K, N21K, L27P, N35R, 1K(γE-γE-C20diacid) | 9.97 | 0.36 | 27.7 |
| 9** | N14D, V17R, S19K, N21K, N22K, L27P, N35R, Y37aeF | 2.53 | 0.026 | 94.03 |

| | | | | |
|---|---|---|---|---|
| ** mean values over 3 results | | | | |

### Example 2: Food intake study

Male Sprague-Dawley rats aged 6-7 weeks were housed in individually ventilated cages containing irradiated corncob bedding material, enrichment toys and free access to food and water. Rats were allowed to acclimatise to 12 hours dark followed by 12 hours light reverse cycle for two weeks. Animal body weight was monitored along with clinical signs during the acclimatization period. On day -5, based on body weights all animals were randomized into different groups with each group containing n=8 and being housed individually. Test peptides were administered at 30 nmol/kg as a single subcutaneous dose and effect on food intake compared to a control group administered vehicle only; each group of 8 animals were given a different.

### Test peptide formulations:

Test substances were formulated in 2 mM acetate, 250 mM glycerol, 0.025% Tween-20 and the pH of the formulations were adjusted to 4.

### Acute food intake procedure:

On day 0 (after body weight measurement), food was withdrawn from all the animals and they were dosed with respective formulations subcutaneously at 10 mL/kg dose volume just before dark cycle initiation. Approximately 1 hour post dosing, a measured quantity of fresh feed was provided to all animals and considered as t=0 hour for the purpose of time measurement. The amount of feed remaining (i.e. unconsumed) was measured at 2, 4, 6 and 24 hours. Cumulative food intake at different time points was calculated in Microsoft^{®} Excel using formula: $Feed intake = \left(Initial feed provided-remaining feed available at time point\right)$ Cumulative food intake data was transposed into GraphPad Prism (version 9.1.2) for analysis.

**Results - Table 2: Cumulative Food Intake % Reduction**

| Peptide ID | % Food intake compared to vehicle treated controls | | | |
|---|---|---|---|---|
| | 2h | 4h | 6h | 24h |
| SEQ ID NO: 7 | -3 | -5 | -16 | -15 |
| SEQ ID NO: 8 | -12 | -39 | -34 | -45 |

| | | | | |
|---|---|---|---|---|
| Values given are mean values, averaged across the 8 animals in each group. | | | | |

### Example 3: Pharmacokinetic study

Pharmacokinetic (PK) studies were performed in male Sprague-Dawley (SD) rats, aged 7-8 weeks and weighing 230-270 g. The peptides were administered via both intravenous (i.v.) and subcutaneous (s.c.) routes at a dose of 30 nM/kg. The formulation used for dosing consisted of 2 mM sodium acetate buffer, 250 mM glycerol, and 0.025% Tween-20, with the pH adjusted to 5.0. Blood samples were collected at predetermined time points:
i.v. : 0.083, 0.25, 0.5, 1.0, 2.0, 4.0, 6.0, 24, 48, 72, 96, and 120 hours
s.c.: 0.25, 0.5, 1.0, 2.0, 4.0, 6.0, 12.0, 24, 48, 72, 96, and 120 hours.

Plasma was isolated from the collected blood samples and analyzed using the LC-MS/MS method. The resulting plasma concentration-time data were used to construct PK profiles.

Half-life values for intravenous and subcutaneous administration routes were determined. The fraction (F_{sc}%) of subcutaneous dose detected in the blood plasma (expressed as a % of the blood plasma concentration via the intravenous route) was also determined - this gives a measure of the subcutaneous bioavailability.

**Results - Table 3: Half-life subcutaneous bioavailability data.**

| Peptide ID* | T½ (h) (i.v.) | T½ (h) (s.c.) | F_{sc} (%) |
|---|---|---|---|
| SEQ ID NO: 7 | 10.7±1.8 | 10.6±1.9 | 15.7 |
| SEQ ID NO: 8 | 12.2±1.7 | 15.4±4.2 | 12.2 |

The foregoing disclosure is illustrative only and does not limit the scope of the invention, which is defined only by the appended claims. Variations from the examples above will be envisaged by the skilled person which fall within the claimed scope.

## Claims

1. An amylin 3 receptor agonist, comprising a peptide with at least 91% sequence identity with Formula (Ib):
KCX₃TX₅TCX₈X₉X₁₀RLX₁₃DFLRX₁₈X₁₉SX₂₁X₂₂FGX₂₅X₂₆PX₂₈ X₂₅TNVGSRTX₃₇ Formula (Ib)
wherein:
X₃ is selected from N and R;
X₅ is selected from A and G;
X₈ is selected from A and T;
X₉ is selected from T and V;
X₁₀ is selected from Q and G;
X₁₃ is selected from A and S;
X₁₈ is selected from H, K and R;
X₁₉ is selected from S and K;
X₂₁ is selected from N, P, K and Aib;
X₂₂ is selected from N, NMeN and K;
X₂₅ is selected from A and P;
X₂₆ is selected from I, NMel, N and K;
X₂₈ is selected from S, A and P;
X₂₉ is selected from S and P;
X₃₇ is selected from Y and pxF;
wherein at least one of:
- X₃ is R; and/or
- X₅ is G; and/or
- X₈ is T; and/or
- X₉ is V; and/or
- X₁₀ is G; and/or
- X₁₃ is S; and/or
- X₁₈ is K or R; and/or
- X₁₉ is K; and/or
- X₂₁ is K or Aib; and/or
- X₂₂ is NMeN or K; and/or
- X₂₅ is P; and/or
- X₂₆ is NMel, N or K; and/or
- X₂₈ is A or P; and/or
- X₂₉ is P; and/or
- X₃₇ is pxF.

2. An amylin 3 receptor agonist according to claim 1, wherein at least one of:
- X₁₈ is R or K; and/or
- X₁₉ is K; and/or
- X₂₁ is K or Aib; and/or
- X₂₂ is NMeN or K; and/or
- X₃₇ is pxF, pxF such as azF, aaF or aeF.

3. An amylin 3 receptor agonist according to any preceding claim, wherein X₃ is N, X₅ is A; X₈ is A, X₉ is T, X₁₀ is Q, X₁₃ is A.

4. An amylin 3 receptor agonist according to any preceding claim, wherein X₂₅ is A, X₂₆ is I, X₂₈ is S and X₂₉ is S.

5. An amylin 3 receptor agonist according to claim 4, wherein the peptide has at least 91% sequence identity with Formula (IIb):
KCNTATCATQRLADFLRX₁₈X₁₉SX₂₁X₂₂FGAIPSSTNVGSRTX₃₇ Formula (IIb).

6. An amylin 3 receptor agonist according to claim 4, wherein the peptide has at least 91% sequence identity with Formula (IIIb):
KCNTATCATQRLADFLRHX₁₉SX₂₁X₂₂FGAIPSSTNVGSRTX₃₇ Formula (IIIb).

7. An amylin 3 receptor agonist according to any preceding claim wherein X₁₉ is K and/or X₂₁ is K and/or X₂₂ is K, and/or X₃₇ is pxF, such as azF, aaF or aeF.

8. An amylin 3 receptor agonist according to any preceding claim wherein X₁₉ is K.

9. An amylin 3 receptor agonist according to any preceding claim wherein there is a covalent bridge between the sulphurs of the cysteines at the 2 and 7 positions.

10. An amylin 3 receptor agonist according to any claim 8, wherein the sulphurs are bridged via a covalent bond or a linking unit, wherein the linking unit is C₁₋₃alkylene.

11. An amylin 3 receptor agonist according to any preceding claim, wherein the agonist comprises a fatty acid covalently attached to the peptide.

12. An amylin 3 receptor agonist according to claim 11, wherein the fatty acid is covalently attached to the lysine at position 1 or a lysine at positions 22 or 26 in the peptide.

13. An amylin 3 receptor agonist according to claim 12, wherein the fatty acid is covalently attached to the lysine at position 1 or a lysine at position 26 in the peptide.

14. An amylin 3 receptor agonist according to claim 13, wherein the fatty acid is covalently attached to side chain of the lysine at position 1 in the peptide.

15. An amylin 3 receptor agonist according to any of claims 11 to 14, wherein the fatty acid is a C₁₄₋₂₂ diacid.

16. An amylin 3 receptor agonist according to any of claim 11 to 15 wherein the fatty acid is covalently attached via a linker.

17. An amylin 3 receptor agonist according to claim 16, wherein the fatty acid and linker together are selected from: 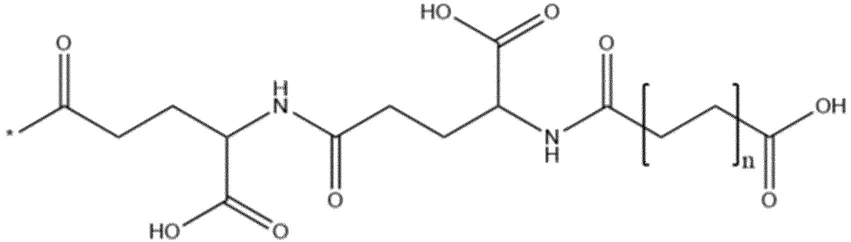 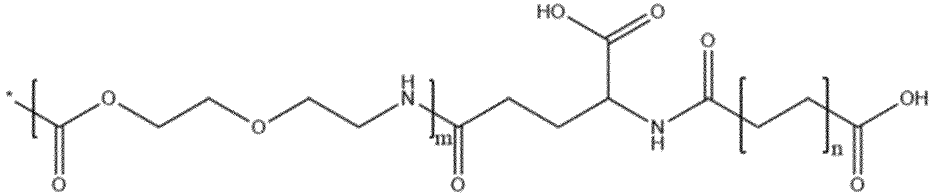 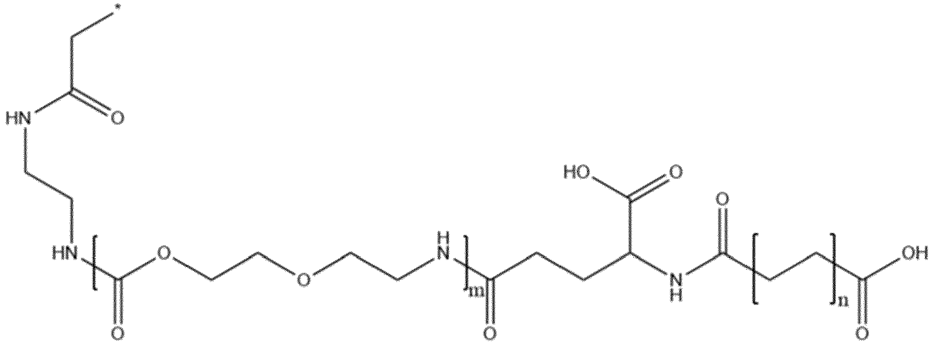 wherein * indicates the point of attachment to the nitrogen in the amino side chain of the lysine n is selected from 12, 14, 16, 18 and 20, and m is 2.

18. An amylin 3 receptor agonist according to any of claims 11 to 14, wherein two or more fatty acids are covalently attached to respective amino acids in the peptide.

19. An amylin 3 receptor agonist according to claim 18, wherein the fatty acids are monoacids, each optionally covalently attached via linkers.

20. An amylin 3 receptor agonist according to claim 1, having a sequence according to any of SEQ ID NO: 3 to 15.

21. A pharmaceutical composition comprising an amylin 3 receptor agonist according to any of claims 1 to 20, and a pharmaceutically acceptable excipient.

22. An amylin 3 receptor agonist according to any of claims 1 to 20, or a pharmaceutical composition according to claim 21, for use in therapy.

23. An amylin 3 receptor agonist according to any of claims 1 to 20, or a pharmaceutical composition according to claim 21, for use in treating or preventing a metabolic disease, disorder and/or condition.

24. An amylin 3 receptor agonist or a pharmaceutical composition for use according to claim 23, for use in treating or preventing obesity.

25. Non-therapeutic use of an amylin 3 receptor agonist according to any of claims 1 to 20, or a pharmaceutical composition according to claim 21, in a method of weight loss, the method comprising administering the amylin 3 receptor agonist or composition to a subject.

26. A method of treating or preventing a metabolic disease, disorder and/or condition in a subject, comprising administering to the subject an amylin 3 receptor agonist according to any of claims 1 to 20, or a pharmaceutical composition according to claim 21.
